# DEMANDE DE BREVET EUROPEEN

(11) **EP 1 340 493 A1**
(43) Date de publication de la demande: **03.09.2003**
(21) Numéro de dépôt: 03354017.0
(22) Date de dépôt: 25.02.2003
(51) Int. Cl.: A61K 9/00, A61K 9/16, A61K 9/20, A61K 47/46, A61K 39/35

(54) **Support solide pour médicament homéopathique extemporane**

(30) Priorité: 28.02.2002 FR 0202516
(71) Demandeur: Betend-dit-Bon, Michel, 74940 Annecy Le Vieux (FR)
(72) Inventeur: Betend-dit-Bon, Michel, 74940 Annecy Le Vieux (FR)
(74) Mandataire: Hecke, G.

(57) **Abrégé**

L'invention porte sur un support solide pour médicament homéopathique préparé en extemporané pour un seul individu et indiqué dans le traitement des maladies allergiques par la méthode dite de désensibilisation spécifique homéopathique.

On prépare des supports ou excipients neutres et inactifs et des excipients biologiquement actifs ; ces excipients seront imprégnés d'un ou de plusieurs allergènes en dilutions homéopathiques. Un excipient neutre et inactif initial devient actif dès qu'il est imprégné d'oeuf de caille qui possède une puissante activité anti-protéasique (anti-inflammatoire et anti-allergique) non spécifique ; la deuxième imprégnation sera faite avec des allergènes en dilutions homéopathiques qui confèrent au comprimé une activité spécifique de l'allergène imprégné (4DH, 5 CH, 7 CH, 9 CH, 12 CH, 15 CH et 30 CH).

Les nouvelles formes galéniques élargissent les indications de la désensibilisation spécifique dans le traitement des maladies allergiques.

## Description

### Domaine technique de l'invention

L'invention concerne un support solide pour médicament homéopathique destiné à la médecine humaine et vétérinaire pour administration per- et sublinguale, ledit support solide étant composé de lactose, de cellulose microcristalline, de croscarmellose sodique et de stéarate de magnésium.

Elle concerne également un médicament homéopathique comportant un support solide.

### État de la technique

La méthode dite de désensibilisation spécifique, notamment utilisée pour le traitement des pathologies allergiques, impose soit des injections sous-cutanées d'allergènes répétées toutes les semaines puis toutes les quinzaines pendant trois à cinq ans, soit l'administration orale ou sublinguale d'allergène liquide et/ou solide. Cette méthode comporte une part d'imprécision pour l'administration d'allergène liquide car une partie des gouttes du produit administré en sublingual est forcément dégluti, les allergènes sublinguaux solides restant le fait exclusif de l'allopathie.

Actuellement, pour effectuer une désensibilisation spécifique on utilise des allergènes (ou antigènes), par voie sublinguale, préparés à la demande pour un seul individu, avec un ou plusieurs allergènes, selon la sensibilisation propre du malade, mise en évidence par le médecin.

Ainsi, le document US6248307 décrit une composition pour une administration orale destinée à traiter les symptômes associés à la ménopause. La composition comporte au moins un médicament homéopathique et un composant à base de plantes.

Le document EP-A1-0315552 décrit des formes galéniques constituées d'extraits d'oeufs de caille réalisés en dilution hahnemanienne à partir d'une solution-mère.

### Objet de l'invention

La présente invention a pour objet un nouveau concept de préparation extemporanée de médicaments homéopathiques solides pour le traitement des maladies allergiques, par la méthode dite de désensibilisation spécifique homéopathique, permettant d'obtenir des produits solides dosés de manière fiable et reproductible avec des allergènes standardisés ou tout autre produit ou médicament homéopathique, par exemple.

L'invention est plus particulièrement définie dans les revendications annexées.

### Description de modes particuliers de réalisation

La présente invention décrira, plus particulièrement, par exemple, et sans restriction dans le choix des médicaments homéopathiques, de nouvelles compositions médicamenteuses pour obtenir un produit utilisable dans le traitement de différentes pathologies et par exemple, les pathologies allergiques, par la méthode dite de désensibilisation spécifique.

La présente invention comporte, par exemple, des granules et des comprimés neutres servant de supports auxquels seront incorporés par pulvérisation ou par tout autre mode de fixation un extrait « d'oeuf de caille » sur lequel, ensuite, par imprégnation ou tout autre mode de fixation, sera fixé un ou des allergènes (ou antigènes) en dilutions homéopathiques.

Par « support neutre » pour utilisation homéopathique, on entend des préparations ayant une consistance solide obtenue à partir du lactose ou saccharose qui peuvent être, par exemple, des granules, des comprimés, des globules ou des poudres.

Par « allergène » ou « antigène », on entend toute substance capable de provoquer dans un organisme humain ou animal une réaction spécifique qui va induire dans certaines conditions des manifestations cliniques allergiques.

Par « oeuf de caille », on entend par exemple, l'oeuf entier de l'espèce aviaire coturnix coturnix japonica et notamment la souche B. Mina Japonica.

Par « extrait », on entend toute fraction ou partie biologiquement active (l'oeuf de caille étant considéré comme un produit naturel), obtenue après différents traitements mécaniques, physiques ou chimiques, ces différents modes de traitement n'étant pas limitatifs, et qui permettent de séparer ou d'enrichir tout constituant dudit oeuf, que ce soit le blanc ou le jaune ou tout autre constituant dudit oeuf. De tels extraits dudit oeuf sont en général obtenus sous forme homogène à l'état sec, par séchage par exemple, ce qui permet d'obtenir une poudre.

Par « homogénat », on entend un mélange aqueux de blancs et de jaunes d'oeufs de caille plus du blanc, dans des proportions contrôlées et reproductibles dont on utilisera un extrait dans lequel la proportion de blancs par rapport aux jaunes pourra être par exemple de 2 pour 1.

Par « blanc » d'oeuf de caille, on entend exclusivement le blanc séparé du jaune que l'on utilisera comme constituant actif dudit oeuf de caille dans des proportions contrôlées et reproductibles dont on prélèvera une partie aliquote, par exemple, selon les modalités de fabrication.

Par « jaune » d'oeufs de caille, on entend exclusivement le jaune séparé du blanc que l'on utilisera par exemple dans des proportions contrôlées et reproductibles et dont on prélèvera une partie aliquote, selon les modalités de fabrication.

Par « arôme », on entend, par exemple, des produits comme la vanille, la framboise, la fraise, la banane ou l'orange, qui seront ajoutés au comprimé sublingual de manière à améliorer l'acceptabilité du produit fini.

Selon l'état antérieur, on connaît le document EP-A1-315 552 qui décrit une composition médicamenteuse à base d'oeufs de caille indiquée dans les maladies allergiques, composition administrée par voie per- et sublinguale sous la forme galénique d'un comprimé à délitement immédiat, à une dilution hahnemannienne, par exemple, de 3 DH (troisième dilution décimale hahnemannienne).

Ce comprimé est une composition médicamenteuse à base d'oeufs de caille, comprenant un support divisé adapté à un délitement immédiat per- et sublingual du principe actif ; cette composition est obtenue par dilutions hahnemanniennes d'une solution-mère (ou souche) dans de l'eau purifiée ou distillée, contenant un mélange de jaunes et de blancs d'oeufs de caille et une quantité définie de blancs seuls incorporés dans ledit support.

Le support est choisi parmi les poudres, les gélules, les globules et les comprimés ou toute autre forme pharmaceutique acceptable et en ce que les oeufs de caille appartiennent à la souche B. Mina et, caractérisée en ce que le produit final est un comprimé à usage per- et sublingual obtenu par imprégnation ou compression ou par tout autre procédé.

Ce comprimé à délitement immédiat ou tout autre forme de support actif marquait jusqu'alors l'état de la technique mais il n'est pas adapté à l'utilisation d'une désensibilisation extemporanée. En effet, l'homéopathie impose une standardisation du support biologiquement actif qui, en la matière, se trouve être 4CH. Cette dilution permet de réaliser d'une façon constante et reproductible, après avoir été elle même fixée sur un noyau de saccharose par exemple, l'imprégnation d'un ou plusieurs allergènes ou antigènes sans altérer la revendication thérapeutique homéopathique. Les allergènes (ou antigènes) pourront être un ou des acariens, un ou des pollens, une ou plusieurs moisissures (ces exemples ne sont pas limitatifs), et seront choisis à une dilution homéopathique précise, les dilutions du ou des allergènes (ou antigènes) étant croissantes de 4DH à 30CH par exemple, et plus particulièrement de 4 CH (quatrième dilution centésimale hahnemannienne), ou 5 CH, 7 CH, 9 CH, 12 CH, 15 CH, 30 CH ou de toutes autres dilutions homéopathiques par exemple.

Selon la présente invention, le support ou excipient pourra être :
- un support inerte neutre, granules ou comprimés, par exemple, auquel on incorporera, par imprégnation ou tout autre mode de fixation, un ou des allergènes (ou antigènes) en dilutions homéopathiques,
- un support inerte neutre ou comprimé auquel sera incorporé par imprégnation par exemple un extrait d'oeuf de caille qui pourra être par exemple :
   ■ un mélange homogène de blancs et de jaunes d'oeufs de caille de souche B. Mina d'un poids moyen de 0,400 mg par exemple, auquel on ajoutera du blanc seul selon un poids moyen de 0,400 mg par exemple, mélange dans lequel la proportion de blanc par rapport au jaune sera de 2 pour 1 par exemple ou,
   ■ du blanc d'oeufs de caille seul d'un poids moyen de 0,800 mg par exemple.

Ce comprimé neutre imprégné d'oeufs de caille pourra servir de support à différents médicaments homéopathiques dont les allergènes, présente une activité biologique et pharmacologique qui lui sont conférés par ledit oeuf de caille, à l'opposé des supports inactifs neutres utilisés habituellement en homéopathie, granules, globules, comprimés ou poudres, par exemple.

Différemment, le comprimé selon l'invention, composé d'un mélange homogène de blancs d'oeufs de caille seul ou de blancs et de jaunes d'oeufs de caille va servir de support aux allergènes (ou antigènes) en dilutions homéopathiques, ou tout autre médicament homéopathique. Le comprimé selon l'invention possède des propriétés biologiques et pharmacologiques qui lui confèrent une activité propre :
- le jaune d'oeuf de caille de souche B. Mina Japonica a une action de transporteur (activité liposomiale) c'est-à-dire qu'il sert de vecteur aux principes actifs pour les transporter vers les sites récepteurs de l'organisme. Par ailleurs, il possède une teneur importante en acides aminés comme, l'histidine, la lysine, le tryptophane et la méthionine, qui potentialisent l'absorption par l'organisme de certains oligo-éléments comme le zinc, lequel accroît les défenses immunitaires. contient une faible quantité de cholestérol,
- le blanc d'oeuf de caille de souche B. Mina possède plusieurs propriétés pharmacologiques liées à sa composition, comme par exemple une activité anti-protéasique liée à l'ovomucoïde et à l'ovoinhibiteur caractérisé par une activité anti-inflammatoire puissante mais non spécifique, la plus puissante des blancs des oeufs de toutes les autres espèces aviaires.

Outre l'activité anti-inflammatoire (anti-protéasique et anti-allergique), le blanc d'oeuf de caille possède une activité anti-allergique liée à l'inhibition de la libération des médiateurs chimiques responsables des manifestations allergiques, lesquels médiateurs sont stockés dans les cellules immunocompétentes comme les mastocytes, les basophiles et les cellules de Langerhans par exemple.

Outre ces propriétés particulières, le blanc d'oeuf de caille contient également de nombreux acides aminés comme l'histidine, la lysine, le tryptophane et la méthionine, qui potentialisent l'absorption de différents oligo-éléments par l'organisme dont le zinc qui augmente les défenses immunitaires de l'organisme ; le mélange homogène de jaunes et de blancs d'oeufs de caille induit un état de tolérance par voie orale dès qu'il entre en contact avec la muqueuse buccale et la muqueuse sublinguale par exemple, ce qui a pour effet de libérer des facteurs suppresseurs comme le TGF-β1 qui bloque la synthèse des anticorps de la classe des IgE, potentialisée par l'interleukine 4 (IL4); le blanc d'oeuf de caille ne contient pas de cholestérol.

Il n'était pas évident pour l'Homme de l'art d'utiliser un support solide à usage per-et sublingual imprégné d'une fraction d'oeufs de caille constituée exclusivement de blanc et aromatisée (ou toute autre forme galénique utilisant ces ou cette fraction), par exemple comme support ou excipient biologiquement et pharmacologiquement actif ; ce support actif non spécifique servira à fixer selon les techniques d'imprégnation ou de compression différents médicaments homéopathiques dont les allergènes en dilutions homéopathiques, obtenant ainsi des préparations extemporanées spécialement préparées pour un individu selon la prescription du médecin ; le comprimé inerte initial après une première imprégnation d'oeuf de caille sera doté de propriétés anti-protéasiques, (anti-inflammatoires et anti-allergiques) non spécifiques, et après une deuxième imprégnation avec un allergène déconcentré, aura acquis en plus, des propriétés anti-allergiques spécifiques, par exemple, du ou des allergènes (ou antigènes) déconcentrés imprégnés.

Selon l'invention, la désensibilisation homéopathique spécifique, dénuée de tout effet secondaire, élargit considérablement les indications de ce mode de traitement des maladies allergiques par sa facilité d'emploi et une excellente adhésion au traitement par le malade ; ces nouvelles possibilités thérapeutiques n'étaient pas prévisibles auparavant. Les principales indications sont par exemple, l'asthme, la trachéite spasmodique, la rhinite pollinique, la rhinite perannuelle, l'urticaire, l'oedème de Quincke, l'eczéma atopique, les conjonctivites, les allergies alimentaires, ces exemples n'étant pas limitatifs.

Les allergènes les plus fréquents sont, par exemple, les pollens, les acariens, les moisissures domestiques et atmosphériques, les squames et les poils d'animaux, certains aliments, le latex, les venins d'insectes piqueurs, les médicaments et les vaccins. Cette liste n'est pas limitative car les nombreuses substances de l'environnement sont susceptibles de devenir agressives pour l'organisme et de se transformer en allergène ou antigène.

Outre les allergènes ou antigènes énumérés ci-dessus il faut notamment citer les allergènes microbiens, mycosiques, parasitaires et viraux, par exemple.

### I/ Obtention d'un granule ou support neutre et inerte :

Les supports neutres et inertes utilisés sont par exemple des granules, des globules, des comprimés ou des poudres. Ces différents supports ou excipients sont imprégnés, selon l'invention, d'une dilution homéopathique, par exemple, d'un ou de plusieurs allergènes (ou antigènes) ou tout autre médicament homéopathique en utilisant une technique d'agitation puis un séchage à une température toujours inférieure à 40°C; selon l'invention les granules qui seront imprégnés d'une dilution homéopathique d'allergènes le seront dans une proportion par exemple, de 1% v/m (ainsi que pour les granules et les poudres), par exemple.

Ces granules sont constituées selon l'invention par :
- un produit de charge, par exemple le lactose,
- un produit liant, par exemple, la cellulose microcristalline,
- un produit de désagrégation, par exemple, la croscarmellose sodique,
- un produit lubrifiant, par exemple, le stéarate de magnésium.

Les granules ont, par exemple, une masse moyenne de 50 mg chacun. Ils ne seront pas aromatisés.

### II/ Exemple de formulation d'un granule pour imprégnation d'un ou plusieurs allergènes (ou antigènes) :

| | |
|---|---|
| Lactose | 39 mg |
| Cellulose microcristalline | 9 mg |
| Croscarmellose sodique | 1.75 mg |
| Stéarate de magnésium | 0.25 mg |

### III / Procédé de préparation d'un granule pour imprégnation d'un ou de plusieurs allergènes (ou antigènes) :

Les granules ou supports inertes et neutres ont une consistance solide et sont préparés à partir de lactose par exemple ou de saccharose, voire un mélange des deux ; selon l'invention, on prend par exemple du lactose ; après imprégnation du granule par le principe actif, la voie d'administration sera sublinguale. La fabrication de ces granules est standardisée. On a recours à des techniques voisines de l'enrobage en turbine. Une proportion conséquente de cristaux calibrés forme les noyaux initiaux ; ils sont placés dans une turbine à dragéifier, puis ils sont montés lentement par succession de couches de lactose par exemple. Le lactose est déposé par pulvérisation d'un sirop à chaud sur la masse en mouvement une fois répartie de manière uniforme le sirop modifie la masse qui devient collante et se mobilise moins facilement dans la turbine. Tout de suite après la pulvérisation, on poudre à nouveau la masse obtenue avec du lactose pour absorber l'humidité permettant ainsi au noyau de reprendre leur mouvement rotatoire. On laisse alors tourner la turbine sous un flux d'air chaud pour éliminer l'excédent d'eau contenu dans l'enrobage pendant deux à trois minutes. Dès que la première couche d'enrobage est cristallisée, on en applique une seconde selon les mêmes modalités et on recommence les opérations en procédant par alternance : pulvérisation, poudrage, séchage. Ces opérations sont répétées plusieurs fois jusqu'à ce que l'on obtienne des granules. Pendant le montage on procède à des tamisages pour obtenir, à la fin, une homogénéité de taille des granules. Pour avoir une bonne conservation de ces granules, on procède à un séchage complémentaire qui se fait à 35°C pendant quelques heures dans une étuve à plateaux.

Puis, pour contrôler la bonne reproductibilité de l'imprégnation des allergènes en dilutions homéopathiques sur le granule d'un lot à un autre, on vérifiera la capacité d'imprégnation qui dépend de la porosité desdits granules, et on utilisera un porosimètre à pénétration de mercure qui permet d'apporter toutes les précisions nécessaires.

### IV/ Obtention d'un comprimé ou support neutre et inerte pour imprégnation d'un ou de plusieurs allergènes (ou antigènes):

Selon l'invention, les comprimés ou supports neutre et inertes seront imprégnés de dilutions homéopathiques d'allergènes (ou antigènes) ou de tout autre médicament homéopathique selon la méthode décrite ci-dessus pour les granules par exemple, l'imprégnation du comprimé par un ou plusieurs allergènes (ou antigènes) se fera, par exemple, dans une proportion de 2% v/m.

Les comprimés seront constitués par exemple par :
- un produit de charge, par exemple, le lactose,
- un produit liant, par exemple, la cellulose microcristalline,
- un produit de désagrégation par exemple, la croscarmellose sodique,
- un produit lubrifiant, par exemple, le stéarate de magnésium.

Selon l'invention, les comprimés auront, par exemple, un poids moyen de 100mg et ne seront pas aromatisés.

### V / Exemple de formulation d'un comprimé neutre et inerte pour imprégnation :

| | |
|---|---|
| Lactose | 78 mg |
| Cellulose microcristalline | 18 mg |
| Croscarmellose sodique | 3.50 mg |
| Stéarate de magnésium | 0.50 mg |

### VI/ Procédé de préparation d'un comprimé neutre et inerte :

Les comprimés inertes selon l'invention, seront préparés par compression directe en leur ajoutant des substances dites auxiliaires, comme par exemple un agent de désagrégation et un lubrifiant. Leur administration se fera, selon l'invention, préférentiellement par voie sublinguale.

Selon l'invention le ou les allergènes (ou antigènes) en dilutions homéopathiques, seront fixés sur les comprimés par imprégnation.

### VII/ Obtention d'un excipient à base d'homogénat d'oeuf de caille pour imprégnation d'un ou de plusieurs allergènes (ou antigènes) :

Cet excipient va servir de support à un ou à plusieurs allergènes (ou antigènes) donnés, qui seront incorporés par imprégnation, par exemple.

L'homogénat comprend du jaune et du blanc d'oeuf de caille plus du blanc seul, tel que décrit dans le document EP-A1-315 552 : la préparation utilise des oeufs de caille de toutes espèces et provenant par exemple la souche B. Mina Japonica produits par un élevage placé sous surveillance sanitaire et vétérinaire. Les oeufs de caille sont préalablement traités de façon à supprimer toute trace de germes pathogènes sur les coquilles, par exemple par immersion dans un ou plusieurs bains de solutions antiseptiques. Les oeufs sont cassés mécaniquement et déversés dans une cuve maintenue sous agitation ou par exemple vidés par aspiration après perforation de la coquille. Après avoir séparé le blanc et le jaune des coquilles on procède à une filtration de manière à obtenir un mélange de blancs et de jaunes d'oeufs de caille dans lequel on aura plus de blancs que de jaunes d'oeufs de caille.

Selon l'invention, cet extrait d'oeufs de caille sera incorporé à un excipient qui lui conférera des propriétés de délitement sublingual immédiat et qui lui assurera une excellente stabilité. Cet excipient, qui va devenir le support des allergènes en dilutions homéopathiques, est constitué par exemple par les produits suivants :
- un produit de charge, par exemple, le lactose,
- un produit liant, par exemple, la cellulose microcristalline,
- un produit de désagrégation par exemple, la croscarmellose sodique,
- un produit lubrifiant, par exemple, le stéarate de magnésium,
- un arôme d'orange, par exemple.

Des exemples de formulation, selon l'invention, sont décrits ci-dessous:

### VIII / Exemple de formulation de l'excipient à base d'oeuf de caille pour imprégnation d'allergènes homéopathiques :

| | |
|---|---|
| Homogénat d'oeufs de caille (blanc et jaune) | 0,400 mg |
| Blanc d'oeuf de caille seul | 0,400 mg |
| Lactose | 150 mg |
| Cellulose microcristalline | 24 mg |
| Croscarmellose sodique | 4 mg |
| Stéarate de magnésium | 0.980 mg |
| Arôme orange | 10 mg |

### IX/ Procédé de préparation du support ou des allergènes :

Un lactose microgranulé est imprégné d'oeufs de caille dont on va préalablement mélanger intimement le blanc et le jaune. Cette imprégnation peut s'effectuer, par exemple, sur 120 g de lactose. On ajoute ensuite à nouveau une quantité de l'ordre de 120 g de lactose et on mélange intimement pour imprégner de manière la plus homogène la totalité du lactose. On répète cette opération jusqu'à ce que l'on obtienne un volume égal au 1/10^{ème} de la quantité totale. Entre chaque opération de déconcentration, on utilise la dynamisation décrite par Hahnemann.

Puis on pèse le solde de lactose, la cellulose microcristalline et la croscarmellose sodique. On incorpore ce mélange au lactose imprégné et on homogénéise durant une demi-heure. A la fin, on pèse et on ajoute le stéarate de magnésium et on mélange une dernière fois durant cinq minutes.

Après séchage durant une nuit à 40°C, les microgranules servent à obtenir des comprimés, par compression directe par exemple. L'arôme orange est introduit à froid par imprégnation.

### X/ Obtention d'un excipient à base de blanc d'oeuf de caille seul :

On procède comme décrit ci-dessus avec l'homogénat mais après la séparation du jaune et du blanc on ne conserve exclusivement que le blanc ; on procède à une filtration et l'on obtient une filtrat ne contenant que du blanc d'oeuf de caille ; ce filtrat va être incorporé à un excipient qui lui donne ses propriétés de délitement immédiat par voie per- et sublinguale et qui lui assure une excellente stabilité ; cet excipient qui va devenir le support des allergènes est constitué, par exemple, par les produits suivants :
- un produit de charge, par exemple, le lactose,
- un produit liant, par exemple, la cellulose microcristalline,
- un produit de désagrégation par exemple, la croscarmellose sodique,
- un produit lubrifiant, par exemple, le stéarate de magnésium,
- un arôme d'orange, par exemple.

Des exemples de formulation, selon l'invention, sont décrits ci-dessous.

### XI/ Formulation de l'excipient à base de blanc d'oeuf de caille seul:

| | |
|---|---|
| Blanc d'oeuf de caille | 0,800 mg |
| Lactose | 150 mg |
| Cellulose microcristalline | 24 mg |
| Croscarmellose sodique | 4 mg |
| Stéarate de magnésium | 0.980 mg |
| Arôme orange | 10 mg |

### XII/ Procédé de préparation du support de l'allergène à l'aide du blanc d'oeuf de caille seul :

Un lactose microgranulé est imprégné de blanc d'oeuf de caille seul ; cette imprégnation peut s'effectuer, par exemple, sur 120 g de lactose ; on ajoute ensuite, de nouveau, une quantité de l'ordre de 120 g de lactose et on mélange intimement pour imprégner de façon très homogène la totalité du lactose ; on répète cette opération jusqu'à l'obtention d'un volume égal au 1/10^{ème} de la quantité totale. Entre chaque opération de déconcentration, on utilise la dynamisation décrite par Hahnemann.

Puis on pèse le solde de lactose, la cellulose microcristalline et la croscarmellose sodique. On incorpore ce mélange au lactose déjà imprégné et on homogénéise pendant une demi-heure. A la fin, on pèse et on ajoute le stéarate de magnésium et on mélange une dernière fois pendant cinq minutes.

Le séchage se fait pendant une nuit à 40°C, les microgranules servant à obtenir des comprimés, par compression directe par exemple.

L'arôme orange par exemple est introduit à froid par imprégnation.

### XIII/ Obtention d'un ou des allergènes (ou antigènes) en dilutions homéopathiques avant l'incorporation dans l'excipient :

Par allergène (ou antigène), on entend une préparation issue de l'extraction des constituants actifs de substance animale ou végétale à l'aide d'un milieu adéquat. On peut se servir, par exemple, pour les substances solubles, du bicarbonate d'ammonium à 4% qui reproduit les conditions d'extraction que l'on peut réaliser au niveau des muqueuses humaines.

Les allergènes (ou antigènes) que l'on utilise usuellement, sont standardisés. On peut donc recourir aux allergènes (ou antigènes) qui servent au diagnostic « in vivo » pour les tests cutanés ainsi qu'aux allergènes utilisés dans le traitement dit de désensibilisation allopathique des maladies allergiques provoquées par ces mêmes allergènes.

La présente invention a recours à des allergènes aqueux (ou antigènes) qui sont purs et dilués, par exemple du 1/10^{ème} au 1/10 000 000ème.

La préparation des allergènes (ou antigènes) homéopathiques peut être basée sur la méthode des dilutions homéopathiques selon la méthode d'Hahnemann, dite des flacons séparés.

L'extrait d'allergène (ou antigène) que l'on va imprégner sur le comprimé d'homogénat (jaune et blanc d'oeuf de caille plus du blanc seul) par exemple, ou sur le comprimé de blanc seul (un extrait d'acarien par exemple), est fourni pur et lyophilisé. Le véhicule utilisé pour les dilutions est l'eau distillée ou du sérum physiologique, par exemple.

On prépare une série de flacons et de bouchons neufs lavés à l'eau distillée stérile et séchés en nombre correspondant au numéro de la dilution prévue, décimale ou centésimale, par exemple.

Outre le premier flacon qui va contenir le ou les allergènes (ou antigènes), les autres flacons qui vont servir aux dilutions successives sont d'abord remplis de véhicule, par exemple de l'eau distillée, pour éviter l'absorption sur les parois ; les opérations de succussion (la succussion consiste à secouer le flacon cent fois), permettant d'assurer l'homogénéité du mélange ou sa dynamisation.

Dans le premier flacon d'allergène (ou antigène) dilué au 1/100ème, on complète à dix parties égales, en volume, avec 9 ml de l'eau distillée afin d'obtenir une dilution décimale hahnemannienne (DH) ou avec 99 ml d'eau distillée pour obtenir une dilution centésimale (CH).

On procède de la même manière jusqu'à la dilution souhaitée.

Pour un allergène (ou antigène) donné, par exemple, l'acarien dermatophagoides pteronyssinus, par exemple, on prépare des dilutions de 3 CH, 5 CH, 7 CH, 9 CH, 12 CH, 15 CH, 30 CH, par exemple. C'est à partir d'une progression croissante que la désensibilisation spécifique sera conduite. La pharmacopée, dans Xème édition de 1983, ne mentionne aucune limitation de dilutions.

L'allergène (ou antigène) lyophilisé et pur peut servir aussi, par exemple, à obtenir des dilutions allopathiques. L'allergène pur et dilué de 10 en 10, par exemple, jusqu'à 1/10⁻⁷. On peut le fixer par imprégnation, par exemple, sur le support actif d'oeuf de caille (jaune et blanc d'oeuf de caille plus une autre addition de blanc seul) ou sur le support actif de blanc d'oeuf de caille seul.

### XIV/ Méthode de fabrication des comprimés d'allergènes (ou antigènes) en dilutions homéopathiques :

Selon l'invention, toutes les précautions nécessaires seront prises pour chacune des dilutions afin que la quantité de solvant qui contient l'allergène (ou antigène) reste constante et pour que toutes les imprégnations soient réalisées de manière homogène et reproductible.

On utilise une turbine et un injecteur du type « spray doseur » et on procède à l'imprégnation des excipients :
- granules neutres et inertes,
- comprimés neutres et inertes,
- comprimé inerte imprégné d'un mélange homogène de jaune et de blanc d'oeuf de caille, ledit mélange étant d'un poids moyen de 0.400 mg par exemple, et de blanc d'oeuf de caille seul, d'un poids moyen de 0.400 mg par exemple de sorte que le mélange jaune et blanc plus blanc, soit d'un poids moyen de 0.800 mg par exemple ; la technique d'imprégnation fait appel à la multi-imprégnation ou à l'imprégnation fractionnée afin de garantir une parfaite répartition du principe actif et une excellente stabilité.
- Comprimé inerte imprégné de 0.800 mg en poids moyen de blanc d'oeuf de caille seul ; la technique d'imprégnation fait appel à la multi-imprégnation.

Entre chaque imprégnation, le séchage est effectué par un passage d'air forcé et desséché dans une chambre où une dépression obtenue par la différence de débit qui existe entre ce passage d'air et un extracteur très puissant.

Selon une caractéristique importante de l'invention, cette opération de séchage sera toujours effectuée à une température inférieure ou égale à 40°C.

La dernière imprégnation sera une imprégnation dite « protectrice », du type « dragéification ».

La dernière phase opératoire consiste à mettre les comprimés dans des tubes doses, par exemple, et à déterminer le conditionnement. Chaque produit sera numéroté selon les doses ou les dilutions homéopathiques qui sont croissantes (3 DH ou 3 CH, 5 DH ou 5 CH, 7 DH ou 7 CH, par exemple).

Il est bien évident que selon l'invention, les exemples de déconcentrations cités ne sont pas limitatifs, comme ne le sont pas les modes de dilutions cités auparavant à titre d'exemple ; ces concentrations, modes de dilutions, peuvent évidemment être préparés extemporanément sur demande pour un seul individu par exemple.

## Revendications

1. Support solide pour médicament homéopathique destiné à la médecine humaine et vétérinaire pour administration per- et sublinguale, ledit support solide étant composé de lactose, de cellulose microcristalline, de croscarmellose sodique et de stéarate de magnésium, support solide **caractérisé en ce qu'**il est imprégné par au moins une première quantité de blanc d'oeuf de caille, de manière à transformer ledit support inactif en un support actif susceptible de recevoir un allergène ou un antigène ou un mélange d'allergènes ou d'antigènes, en dilutions adaptées à une désensibilisation extemporanée homéopathique.

2. Support solide selon la revendication 1, **caractérisé en ce que** la première quantité de blanc d'oeuf de caille est de l'ordre de 0,400mg ou de 0,800mg.

3. Support solide selon l'une des revendications 1 et 2, **caractérisé en ce qu'**il est imprégné par une seconde quantité d'un mélange homogène de blanc d'oeuf de caille et de jaune d'oeuf de caille.

4. Support solide selon la revendication 3, **caractérisé en ce que** lesdites première et seconde quantités du blanc d'oeuf de caille et du mélange homogène sont chacune de l'ordre de 0,400mg.

5. Support solide selon l'une quelconque des revendications 1 à 4, **caractérisé en ce que** les oeufs de caille sont de la souche B.Mina de l'espèce coturnix coturnix japonica.

6. Support solide selon l'une quelconque des revendications 1 à 5, **caractérisé en ce que** le support solide imprégné est aromatisé à l'orange, la fraise, la framboise, la banane, ou la vanille.

7. Support solide selon l'une quelconque des revendications 1 à 6, **caractérisé en ce que** le support solide est sous forme de granule, de comprimé, de poudre, de gélule ou de globule.

8. Médicament homéopathique comportant un support solide selon l'une des revendications 1 à 7, **caractérisé en ce que** le support solide est imprégné d'allergène ou d'un antigène ou d'un mélange d'allergènes ou d'antigènes, en dilutions homéopathiques croissantes de 4DH à 30CH.

9. Médicament homéopathique selon la revendication 8, **caractérisé en ce que** l'allergène est choisi parmi les acariens, les pollens, les moisissures domestiques et atmosphériques, les squames, les poils d'animaux, les aliments, le latex, les venins d'insectes piqueurs, les médicaments, les vaccins, les allergènes microbiens, les allergènes mycosiques, les allergènes parasitaires et les allergènes viraux.

10. Médicament homéopathique selon l'une des revendications 8 et 9, **caractérisé en ce qu'**avant imprégnation, l'allergène ou l'antigène servant de souche est lyophilisé, pur et peut servir à préparer des dilutions allopathiques de 10 en 10 jusqu'à 1/10⁻⁷, les dilutions étant destinées à être imprégnées sur le support solide.
